**Europäisches Patentamt**

(19) **European Patent Office**

**Office européen des brevets**

(11) Veröffentlichungsnummer: **0 183 048**
**B1**

(12) # EUROPÄISCHE PATENTSCHRIFT

(45) Veröffentlichungstag der Patentschrift:
22.06.88

(21) Anmeldenummer: **85113413.0**

(22) Anmeldetag: **22.10.85**

(51) Int. Cl.⁴: **C 12 Q 1/28** // C12Q1/30

(54) **Verfahren zur Bestimmung der Peroxidase.**

(30) Priorität: **22.10.84 DE 3438683**

(43) Veröffentlichungstag der Anmeldung:
**04.06.86 Patentblatt 86/23**

(45) Bekanntmachung des Hinweises auf die Patenterteilung:
**22.06.88 Patentblatt 88/25**

(84) Benannte Vertragsstaaten:
**AT BE CH DE FR GB IT LI LU NL SE**

(56) Entgegenhaltungen:
**EP - A - 0 121 254**

**CHEMICAL ABSTRACTS, Band 99, Nr. 5, 01. August 1983, Seite 225, Spalte 1, Zusammenfassungsnr. 34881x, Columbus, Ohio, US; W.D. GEOGHEGAN: "Adaptation of the Ngo-Lenhoff peroxidase assay for solid phase ELISA", & J. IMMUNOL. METHODS 1983, 60(1-2), 61-68**

(73) Patentinhaber: **Boehringer Mannheim GmbH, Sandhofer Strasse 112-132, D-6800 Mannheim-Waldhof (DE)**

(72) Erfinder: **Wehner, Rainer, Dr. rer. nat., Buchendorfer Strasse 16, D-8035 Gauting (DE)**
Erfinder: **Lenz, Helmut, Dr. rer. nat., Waldschmidtstrasse 7, D-8132 Tutzing (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

**Beschreibung**

Die Bestimmung von Peroxidase ist auf verschiedenen technischen Gebieten von Bedeutung. In den letzten Jahren hat dabei insbesondere die zunehmende Verwendung von Peroxidase als Markierungsenzym im Rahmen der sogenannten Enzymimmunbestimmung (EIA), welche die früher übliche Radioimmunbestimmung (RIA) in beträchtlichem Umfang verdrängt hat, grosse Bedeutung erlangt. Die Enzymimmunbestimmung von Haptenen, Antigenen und Antikörpern stellt ein ausserordentlich empfindliches Verfahren dar, welches daher auch entsprechend hohe Anforderungen an die Genauigkeit der Bestimmung der dabei verwendeten Markierungsenzyme stellt. Bei der sehr häufigen Verwendung von Peroxidase als Markierungsenzym in diesem Rahmen erfolgt die Enzymaktivitätsbestimmung häufig nach der fixed-time-Methode durch Extinktionsmessung. Das Verfahren beruht im prinzip darauf, dass die Peroxidase (POD) ein geeignetes Chromogen in Gegenwart eines Peroxids unter Farbbildung oxidiert, wobei die Farbbildungsgeschwindigkeit der Aktivität bzw. Menge der POD entspricht. Bei der «fixed-time»-Methode wird nach einer definierten Reaktionszeit nach Zugabe des Substrates (meist 30 bis 60 Minuten) die gebildete Farbe gegen einen Leerwert gemessen. Da die Farbreaktion zu diesem Zeitpunkt im allgemeinen noch nicht abgeschlossen ist, muss gewährleistet sein, dass für alle Standards und Proben gleiche Inkubationszeiten eingehalten werden. Dies erfordert, dass sowohl die Substratzugabe als auch die Extinktionsmessung in einem exakt festgelegten Abstand von in der Regel 10 bis 15 Sekunden zwischen den einzelnen Teströhrchen vorgenommen werden muss. Dieser, die Handhabung erschwerende Nachteil kann bei Anwendung eines Abstoppmittels für die Reaktion vermieden werden.

Aus der US-PS 4 234 680 ist es bereits bekannt, im oben erläuterten Zusammenhang ein Alkalimetabisulfit als Abstoppmittel einzusetzen. Dieses Mittel hat jedoch den Nachteil, als Reduktionsmittel die bei der Oxidationsreaktion gebildete Farbe anzugreifen und unter Entfärbung zu reduzieren. Dies gilt insbesondere für das besonders als Chromogen im Rahmen der POD-Bestimmung geeignete 2,2'-Azino-di[-3-ethyl-benzthiazolin-sulfonsäure-(6)]-salz, gewöhnlich das Diammoniumsalz (ABTS)®, bei welchem innerhalb kurzer Zeit ein Ausbleichen der Farbe auftritt.

Ein anderes Abstoppmittel für die POD-Reaktion ist Formaldehyd. Hier ist aber ebenfalls die Stabilität der mit dem Mittel versetzten Farblösung unbefriedigend. Hinzu kommt die Geruchsbelästigung und die gesundheitliche Bedenklichkeit von Formaldehyd.

Ein weiteres bekanntes Abstoppmittel für POD ist Oxalsäure. Dieses Mittel hat jedoch eine Verschlechterung der Präzision der Bestimmung zur Folge. Dies gilt besonders dann, wenn zur Enzym-Immunbestimmung mit Antikörpern beschichtete Festkörper (z.B. tubes oder Kugeln) eingesetzt werden, wobei im allgemeinen die an der Wand gebundene POD nach Trennung der flüssigen Phase von der Festphase auf übliche Weise bestimmt wird. Bei Zugabe von Oxalsäure können solche an die Festphase gebundene Proteine jedoch abgelöst und/oder denaturiert werden.

Ein weiteres bekanntes Abstoppmittel ist Natriumazid. Auch hier besteht die Gefahr einer Ausbleichung der Farbe. Ausserdem besteht Explosionsgefahr in Verbindung mit Schwermetallsalzen.

Schliesslich wurden oberflächenaktive Mittel wie sekundäres Alkylsulfat oder Dodecylhydrogensulfat als Abstoppmittel vorgeschlagen. Sie sind jedoch nicht in der Lage, die Farbbildung vollständig zu unterbinden.

Der Erfindung liegt daher die Aufgabe zugrunde, die Nachteile dieser bekannten Abstoppmittel zu beseitigen und ein Verfahren zur Bestimmung von Peroxidase zu schaffen, bei welchem ein Abstoppen der Farbbildung zu einem bestimmten, vorgewählten Zeitpunkt ermöglicht wird, welches sofort wirksam ist und keine nachträgliche Veränderung der zum Zeitpunkt der Zugabe des Abstoppmittels vorhandenen Farbe zur Folge hat.

Gelöst wird diese Aufgabe erfindungsgemäss durch ein Verfahren zur Bestimmung von Peroxidase durch Zusatz eines Peroxids und eines Chromogens und kinetische Messung der bei der Oxidation des Chromogens entstandenen Farbe, wobei die Farbbildung nach einer definierten Zeit durch den Zusatz eines Abstoppmittels gestoppt wird, welches dadurch gekennzeichnet ist, dass man als Abstoppmittel Katalase zusetzt.

Das erfindungsgemässe Verfahren hat eine sofortige Abstoppwirkung auf die Farbbildung, verändert diese jedoch nachträglich nicht, so dass es möglich ist, innerhalb mehrerer Stunden nach dem Abstoppen zu einem beliebigen Zeitpunkt die Messung der gebildeten Farbe vorzunehmen.

Das Verfahren der Erfindung ist für alle im Rahmen der POD-Bestimmung einsetzbaren Chromogene geeignet. Typische Beispiele für geeignete Chromogene sind 2,2'-Azino-di-[3-ethyl-benzthiazolin-sulfonsäure-(6)]-diammoniumsalz (ABTS)®, o-Phenylendiamin, p-Phenylendiamin, m-Aminosalicylsäure, Dianisidin, p-Aminobenzoesäure, Anilin, 4-Aminoantipyrin und dergleichen. Diese Chromogene sind dem Fachmann im Rahmen der POD-Bestimmung bekannt und bedürfen hier keine näheren Erläuterung. Bevorzugt wird ABTS® verwendet. Ebenfalls bevorzugt werden o-Phenylendiamin und 4-Aminoantipyrin.

Die Menge an Katalase, die beim Verfahren der Erfindung zugesetzt wird, ist an sich unkritisch. Sie muss jedoch im Überschuss bezüglich der möglichen POD-Aktivität vorliegen. Im Rahmen von Enzymimmunbestimmung ist die eingesetzte POD-Menge, von der ja nur ein Teil dann bestimmt werden muss, bekannt. Bei Bestimmung von POD in anderem Zusammenhang lässt sich im Zweifel durch einen einfachen Vorversuch die Grössenordnung an POD bestimmen und dann die zur Erzielung eines Katalaseüberschusses erforderliche Menge leicht feststellen. Im allgemeinen werden Mengen von 50 bis 1000 U Katalase pro Bestimmungsansatz in Betracht kommen, für bestimmte Fälle können jedoch kleinere Katalasemengen ausreichen bzw. sind noch grössere Zusätze zweckmässig.

Das Verfahren der Erfindung eignet sich besonders auch dann, wenn die zu bestimmende POD in immunologisch oder chemisch gebundener Form vorliegt. Dies ist beispielsweise der Fall bei Verwendung von POD in Form von Konjugaten, beispielsweise chemisch gebunden an einen Antikörper, ein Antikörperfragment, ein Antigen, eine Globulinfraktion oder dergleichen. Ebenfalls eignet sich das erfindungsgemässe Verfahren sehr gut in Fällen, wo die POD selbst oder über einen mit ihr chemisch verbundenen Liganden immunologisch fixiert vorliegt, welcher an eine Festphase gebunden sein kann. Bei diesen Ausführungsformen der POD-Bestimmung sind die Anforderungen an ein Abstoppmittel besonders kritisch, da Einwirkungen des Abstoppmittels auf die Bindungspartner der POD leicht zu Verfälschungen der Ergebnisse führen können. Natürlich eignet sich die Erfindung erst recht für Bestimmung von freier gelöster POD.

Beim erfindungsgemässen Verfahren kann die Messung der gebildeten Farbe ohne nachteiligen Einfluss auf die Präzision der Bestimmung innerhalb mehrerer Stunden nach Zugabe der Katalase durchgeführt werden. Dies bedeutet eine wesentliche Erleichterung und schaltet verschiedene Fehlermöglichkeiten aus.

Die folgenden Beispiele erläutern die Erfindung weiter.

Beispiel 1
Abstoppen der Farbbildungsreaktion im ABTS®-System

Hierbei wird der Enzym-Test® TSH von Boehringer Mannheim (Best.-Nr. 736082) verwendet. Dieser Test enthält POD chemisch gebunden in Form von Antikörper-POD-Konjugat, welches während der Immunreaktion an die Gefässwand eines Proberöhrchens aus Kunststoff gebunden wird sowie ABTS® als Chromogen und Natriumperborat als Peroxid.

Der Test wird nach der Arbeitsvorschrift des Herstellers mit einem TSH-Standard (50 µU/ml) durchgeführt. Zu untersuchendes Probeserum wird 60 Minuten bei pH 6,9 (Phosphatpuffer) im Proberöhrchen inkubiert, dann wird Antikörper-POD-Konjugat zugesetzt und 60 Minuten inkubiert. Dann werden Perborat und ABTS® in Phosphat-Citratpuffer, pH 4,4 zugegeben. Die Farbbildungsreaktion

$$\text{ABTS}^{\circledR} \text{ (farblos)} \xrightarrow[\text{H}_2\text{O}_2]{\text{POD}} \text{ABTS}^{+\circledR} \text{ (gefärbt)}$$

wird nach 60 Minuten durch Zugabe von 0,1 ml eines Stoppreagenz, bestehend aus:
250 U/ml Katalase
50 mmol/l Acetatpuffer, pH 5,5
2 Gew.-% Triton X-405 (nichtionogenes oberflächenaktives Mittel) abgestoppt. Anschliessend wird die Extinktion der Lösung bei 405 nm über 3 Stunden alle 60 Minuten gemessen. Dabei wird folgende Extinktion (bezogen auf die Zugabe der Lösung (t = 0, E = 100%) erhalten:

Tabelle I

| Zeit (min) | 0 | 60 | 120 | 180 |
|---|---|---|---|---|
| Extinktion | 100% | 99,8% | 100% | 98,5% |

Beispiel 2
Abstoppen der Farbbildungsreaktion im o-Phenylendiamin-System.

Es wird ein handelsüblicher CEA-EIA-Test (ABBOTT Best.-Nr. 586324) verwendet. Dieses Reagenz enthält POD chemisch gebunden als Antikörper-POD-Konjugat, welches während der Immunreaktion an Kunststoffkugeln gebunden wird und als Substrat o-Phenylen-diamin.

Der Test wird, entsprechend der Arbeitsvorschrift des Herstellers, mit 2 CEA-Proben (Konzentration 1 bzw. 5 ng/ml CEA) durchgeführt. Die Oxidation (Farbbildung) von o-Phenylendiamin mit POD und $H_2O_2$ wird nach 30 Minuten durch Zugabe von 2 ml eines Stoppreagenzes gemäss Beispiel 1, welches jedoch im Verhältnis 1:20 mit Wasser verdünnt wurde, abgestoppt. Für die 2 Proben (numeriert in aufsteigender Konzentration) erhält man folgende Extinktionen, die jeweils bei 405 nm gemessen wurden und auf den Zeitpunkt des Abstoppens, t = 0, bezogen wurden

Tabelle II

| Probe | Zeit = 0' | Zeit = 90' |
|---|---|---|
| 1 | 100,0% | 100,0% |
| 2 | 100,0% | 103,1% |

Beispiel 3
Vergleich der Abstoppwirkung von Katalase und Formaldehyd.

Es werden die Reagenzien gemäss Beispiel 1 verwendet. Zum Vergleich wird anstelle des Stoppreagenzes von Beispiel 1 wässrige Formaldehydlösung (25 Gew.-%) verwendet. Das Abstoppen der Farbbildungsreaktion erfolgt mit jeweils 0,2 ml Stoppreagenz. Dabei werden folgende Extinktionen bei 405 nm gemessen (bezogen auf Zeitpunkt des Abstoppens t = 0):

Tabelle III
Formaldehyd als Stoppreagenz

| Zeit | 0' | 60' | 120' | 180' |
|---|---|---|---|---|
| Ext. | 100% | 98,1% | 91,3% | 89% |

Stoppreagenz gem. Beispiel 1
(Katalyse)

| Zeit | 0' | 60' | 120' | 180' |
|------|------|------|------|------|
| Ext. | 100% | 98,8% | 99,8% | 100,1% |

Beispiel 4

Vergleich der Präzision bei Verwendung von Katalase und Oxalsäure als Stoppreagenz

Es werden die Reagenzien gemäss Beispiel 1 verwendet.

Die Durchführung des Versuchs erfolgt analog Beispiel 1, wobei anstelle des dort beschriebenen Stoppreagenzes 0,2 ml einer wässrigen Oxalsäurelösung (7 Gew.-%) zugegeben werden. Der Vergleichsversuch mit Katalase erfolgt entsprechend Beispiel 1. Die Abstoppwirkung von Oxalsäure ergibt sich aus nachstehender Tabelle IV.

Tabelle IV

| Zeit | 0' | 60' | 120' | 180' |
|------|------|------|------|------|
| Ext. | 100% | 112,6% | 117,0% | 114,0% |

Für 10 verschiedene Ansätze mit Katalase bzw. Oxalsäure als Stoppreagenz wurde die Extinktion der Lösung bei 405 nm (Zeitpunkt des Abstoppens t = 0) gemessen. Die Ergebnisse zeigt folgende Tabelle V:

Tabelle V

|  | 0' | 60' | 120' | 180' |
|------|------|------|------|------|
| VK mit Katalase | 1,1 | 0,7 | 1,5 | 2,7 |
| VK mit Oxalsäure | 2,0 | 3,5 | 6,0 | 8,5 |

Sie zeigt, dass die Präzision der Bestimmung mit Oxalsäure als Stoppreagenz deutlich verschlechtert ist.

Beispiel 5

Abstoppen der Farbbildungsreaktion im ABTS®-System (nicht wandgebundenes POD-Konjugat)

Lösung 1: Thyroxin-POD-Konjugat,
Aktivität: 0,6 mU/ml, gelöst in
Phosphatpuffer (40 mM)

Lösung 2: Phosphat-Citrat-Puffer, 100 mmol/l
$H_2O_2$, 3 mmol/l
ABTS®, 1,6 mmol/l

Lösung 3 (Stoppreagenz): Acetatpuffer, 50 mmol/l
Katalase, 250 U/ml
Triton X-405, 2%

Zu 1 ml Lösung 2 werden 8 µl Lösung 1 zugegeben. Die Farbbildungsreaktion

$$\text{ABTS}^{\circledR} \xrightarrow[H_2O_2]{POD} \text{ABTS}^{\oplus}$$

wurde nach 30 Minuten durch Zugabe von 100 µl Lösung 3 abgestoppt und die Extinktion bei 405 nm sofort (T = 0') und nach 60 Minuten gemessen. Die Ergebnisse zeigt Tabelle VI:

Tabelle VI

|  | T = 0' | T = 60' |
|------|------|------|
| $E_{405}$ | 100% | 102% |

**Patentansprüche**

1. Verfahren zur Bestimmung von Peroxidase durch Zusatz eines Peroxids und eines Chromogens und Messung der bei der Oxidation des Chromogens entstandenen Farbe, wobei die Farbbildung nach einer definierten Zeit durch den Zusatz eines Abstoppmittels gestoppt wird, dadurch gekennzeichnet, dass man als Abstoppmittel Katalase zusetzt.

2. Verfahren nach Anspruch 1, dadurch gekennzeichnet, dass man immunologisch oder chemisch gebundene Peroxidase bestimmt.

3. Verfahren nach Anspruch 1 oder 2, dadurch gekennzeichnet, dass man als Chromogen 2,2'-Azino-di-[3-ethyl-benzthiazolin-sulfonsäure-(6)]-salz oder o-Phenylendiamin verwendet.

4. Verfahren nach einem der Ansprüche 1 bis 3, dadurch gekennzeichnet, dass man die Katalase in Form einer gepufferten Lösung, pH 5 bis 7, welche ein nichtionogenes Netzmittel enthält, zusetzt.

5. Verfahren nach einem der vorhergehenden Ansprüche, dadurch gekennzeichnet, dass man als Peroxid Natriumperborat zusetzt.

6. Anwendung des Verfahrens nach Anspruch 1 bis 5 im Rahmen der Enzymimmunbestimmung mit Peroxidase als Markierungsmittel.

**Claims**

1. Process for the determination of peroxidase by addition of a peroxide and of a chromogen and measurement of the colour resulting from the oxidation of the chromogen, whereby the colour formation is stopped after a definite time by the addition of a stop agent, characterised in that one uses catalase as stop agent.

2. Process according to claim 1, characterised in that one determines immunologically or chemically bound peroxidase.

3. Process according to claim 1 or 2, characterised in that one uses a 2,2'-azino-di-[3-ethyl-benzthiazolinesulphonic acid-(6)] salt or o-phenylenediamine as chromogen.

4. Process according to one of claims 1 to 3, characterised in that one adds the catalase in the form of a buffered solution, pH 5 to 7, which contains a nonionic wetting agent.

5. Process according to one of the preceding claims, characterised in that one adds sodium perborate as peroxide.

6. Use of the process according to claim 1 to 5 in the scope of the enzyme immune determination with peroxidase as labelling enzyme.

## Revendications

1. Procédé de dosage de la peroxydase par addition d'un peroxyde et d'un chromogène et mesure de la couleur formée lors de l'oxydation du chromogène, dans lequel la formation de couleur est arrêtée au bout d'un temps défini par addition d'un agent d'arrêt, caractérisé en ce qu'on ajoute comme agent d'arrêt de la catalase.

2. Procédé suivant la revendication 1, caractérisé en ce qu'on dose la peroxydase immunologiquement ou chimiquement liée.

3. Procédé suivant la revendication 1 ou 2, caractérisé en ce qu'on utilise comme chromogène un sel de l'acide 2,2'-azino-di-[3-éthyl-benzothiazoline-sulfonique-(6)] ou l'o-phénylènediamine.

4. Procédé suivant l'une des revendications 1 à 3, caractérisé en ce qu'on ajoute la catalase sous la forme d'une solution tamponnée, pH 5 à 7, qui contient un agent mouillant non ionique.

5. Procédé suivant l'une des revendications précédentes, caractérisé en ce qu'on ajoute comme peroxyde du perborate de sodium.

6. Utilisation du procédé suivant les revendications 1 à 5 dans le cadre du dosage immunoenzymatique avec de la peroxydase comme agent de marquage.